# FASCICULE DE BREVET EUROPEEN

(11) **EP 2 120 590 B1**
(45) Date de publication et mention de la délivrance du brevet: **26.10.2011**
(21) Numéro de dépôt: 08775550.0
(22) Date de dépôt: 13.02.2008
(51) Int. Cl.: A23C 9/12, A61K 35/74, A61P 31/16, A61P 37/02

(54) **UTILISATION DE LACTOBACILLUS CASEI POUR RENFORCER LA PROTECTION INDUITE PAR LA VACCINATION ANTI-GRIPPALE**
VERWENDUNG VON LACTOBACILLUS CASEI ZUR VERSTÄRKUNG DER SCHUTZWIRKUNG DER GRIPPEIMPFUNG
USE OF LACTOBACILLUS CASEI FOR INCREASING THE PROTECTION PROVIDED BY THE INFLUENZA VACCINE

(30) Priorité: 16.02.2007 FR 0701140
(43) Date de publication de la demande: 25.11.2009
(73) Titulaire: Compagnie Gervais Danone, 75009 Paris (FR)
(72) Inventeur: SAMSON-VILLEGER, Sandrine, 69007 Lyon (FR); BOURDET-SICARD, Raphaëlle, F-91120 Palaiseau (FR)
(74) Mandataire: Vialle-Presles, Marie José
(86) Numéro de dépôt international: PCT/FR2008/000181
(87) Numéro de publication internationale: WO 2008/129148

(56) Documents cités:
- WO-A-01/89541
- WO-A-2006/124630
- DE VRESE M ET AL: "Probiotic bacteria stimulate virus-specific neutralizing antibodies following a booster polio vaccination" EUROPEAN JOURNAL OF NUTRITION, STEINKOPFF-VERLAG, DA, vol. 44, no. 7, 1 octobre 2005 (2005-10-01), pages 406-413, XP019383082 ISSN: 1436-6215 cité dans la demande
- YASUI HISAKO ET AL: "Reduction of influenza virus titer and protection against influenza virus infection in infant mice fed Lactobacillus casei shirota" CLINICAL AND DIAGNOSTIC LABORATORY IMMUNOLOGY, vol. 11, no. 4, juillet 2004 (2004-07), pages 675-679, XP002452131 ISSN: 1071-412X
- OGAWA TOMOHIKO ET AL: "Oral immunoadjuvant activity of Lactobacillus casei subsp. casei in dextran-fed layer chickens." THE BRITISH JOURNAL OF NUTRITION FEB 2006, vol. 95, no. 2, février 2006 (2006-02), pages 430-434, XP009089823 ISSN: 0007-1145

## Description

L'invention est relative à l'utilisation de *Lactobacillus casei* pour potentialiser la réponse humorale induite par la vaccination antigrippale chez des sujets âgés et ainsi renforcer la protection contre la grippe après vaccination.

Les virus influenza (virus de la grippe) représentent une cause majeure d'infections respiratoires.

Les épidémies hivernales de grippe affectent 1 à 5% de la population, avec des taux d'infection très importants chez les enfants et des complications mortelles chez les personnes âgées. La vaccination annuelle contre l'influenza est recommandée par les autorités de santé (en accord avec l'OMS) chez les sujets de plus de 65 ans, les personnes vivant en EHPAD (Établissement d'Hébergement pour Personnes Âgées Dépendantes) ou les sujets fragilisés par des pathologies lourdes. Même dans les cas où la vaccination ne prévient pas la maladie, elle permet de diminuer la sévérité, la durée, le risque de complication (surinfection, hospitalisation, mort) associés à la grippe.

Néanmoins, alors que la vaccination antigrippale confère chez la majeure partie des sujets adultes (70% à 90%), un niveau d'anticorps considéré comme protecteur (taux d'anti-Hémagglutinine supérieur ou égal à 40 ; OMS Weekly epidemiological record, N°33, p.283, 19 August 2005) il n'en va pas de même chez les personnes âgées, où ce niveau d'anticorps n'est atteint que chez 30 à 40% des sujets (COX et al., Scand. J. Immunol., 59, 1-15, 2004). Cette réponse sub-optimale des personnes âgées au vaccin anti-grippal est due aux défauts fonctionnels du système immunitaire qui sont liés à l'âge et/ou à l'état physiologique du sujet (DENG et al., J. Immunol., 172, 3437-46, 2004; KANG et al., J. Immunol., 173, 673-81, 2004).

Une façon d'améliorer la réponse immunitaire est d'administrer des substances immuno-stimulantes qui jouent le rôle d'adjuvant. Ainsi, de nouveaux vaccins contenant des adjuvants (comme MF59 qui consiste en des gouttelettes stables de taille < à 250nm, composées de squalène, d'huile métabolisable, et de deux surfactants (polyoxyethylene sorbitan monooleate et sorbitan trioleate) dans une émulsion huile dans eau) commencent à être commercialisés en France pour potentialiser la réponse immunitaire des personnes âgées (BALDO et al., Vaccine, 19, 3472-5, 2001). Par ailleurs, l'alimentation peut moduler les réponses immunitaires. Il a été démontré que les suppléments en zinc, sélénium, certaines vitamines et oligo-éléments pendant 2 à 6 mois pouvaient permettre d'augmenter les réponses immunitaires des personnes âgées en institutions face à la vaccination (ALLSUP et al., J. Am. Geriatr. Soc., 52, 20-4, 2004; BUNOUT et al., JPEN J. Parenter. Enteral. Nutr., 28, 348-54, 2004; BUNOUT et al., JPEN J. Parenter. Enteral. Nutr., 26, 372-6, 2002; CHANDRA, Lancet, 340, 1124-7, 1992; GIRODON et al., Arch. Intern. Med., 159, 748-54, 1999; LANGKAMP-HENKEN et al., J. Am. Geriatr. Soc., 52, 3-12, 2004; PROVINCIALI et al., Age Ageing, 27, 715-22, 1998). Il a été rapporté que certains probiotiques peuvent également moduler la réponse à des vaccins comme celui contre la poliomyélite (DE VRESE et al., Eur. J. Nutr., 44, 406-13, 2005).

Il a été précédemment observé (Demande PCT WO 2001/089541) que l'administration de *Lactobacillus casei* pouvait renforcer la réponse immunitaire cellulaire (médiée par les lymphocytes T) vis-à-vis de divers micro-organismes pathogènes, parmi lesquels le virus de la grippe. En revanche, les éventuels effets de *Lactobacillus casei* sur l'immunité humorale, qui est impliquée dans la protection conférée par la vaccination anti-grippale, demeuraient mal connus.

Les Inventeurs ont entrepris d'étudier l'effet de l'ingestion de *Lactobacillus casei* sur l'évolution des taux d'anticorps sériques spécifiques induits par la vaccination anti-grippale chez les personnes âgées. Ils ont constaté une augmentation de ces taux d'anticorps, aboutissant notamment à une augmentation de la fréquence de séroconversion, et de la fréquence de séroprotection.

On définit comme « séroprotection » la présence, chez un individu, d'anticorps sériques dirigés contre un virus de la grippe, en quantité supérieure ou égale à un seuil de protection. Ce seuil de protection est défini comme un titre en anticorps sériques anti-Hémagglutinine, mesuré en inhibition de l'hémagglutination (IHA), supérieur ou égal à 40. La « fréquence de séroprotection », chez une population, correspond à la proportion de personnes chez lesquelles une séroprotection est observée. Il est généralement admis qu'un taux d'anticorps mesurés IHA et supérieur ou égal à 40 était relié à une protection contre la souche impliquée et retrouvée dans le vaccin (COX N.J. and al., Lancet, 1999:354:1277-82).

On définit ici comme « séroconversion », l'augmentation, chez un individu, de la quantité d'anticorps sériques dirigés contre un virus de la grippe, consécutive à la vaccination contre ledit virus, le niveau d'anticorps post-vaccination étant au moins égal à 4 fois le niveau d'anticorps mesuré avant vaccination. La « fréquence de séroconversion », chez une population, correspond à la proportion de personnes chez lesquelles une séroconversion est observée.

Les Inventeurs ont en outre constaté que les effets positifs de l'ingestion de *Lactobacillus casei* sur l'évolution des taux d'anticorps sériques induits par la vaccination anti-grippale apparaissent particulièrement marqués chez certaines catégories de personnes âgées, à savoir celles présentant le plus faible niveau de dépendance, et celles appartenant au sexe féminin.

Le niveau de dépendance a été défini en utilisant la grille de classification AGGIR (Autonomie Gerontologie Groupes Iso-Ressources ; (VETEL et al., Soins Gerontol., 23-7, 1998). Les groupes 1, 2, et 3 de la grille AGGIR regroupent des personnes fortement dépendantes ; les groupes 4 et 5 regroupent des personnes faiblement dépendantes ; le groupe 6 regroupe les personnes non-dépendantes.

La présente invention a, en conséquence, pour objet l'utilisation d'une souche bactérienne de l'espèce *L. casei* pour la préparation d'une composition administrable par voie orale pour renforcer la protection contre la grippe après vaccination. Elle permet en outre de renforcer l'immunité humorale conférée lors d'une vaccination anti-grippale. Elle permet d'atteindre notamment un taux d'anticorps protecteurs (séroprotection).

Selon un mode de mise en oeuvre préféré de la présente invention, ladite composition est destinée à être administrée à des personnes âgées de 65 ans ou plus recevant une vaccination anti-grippale. De préférence, lesdites personnes sont âgées d'au moins 70 ans.

De manière particulièrement avantageuse, ladite composition est destinée à être administrée à des personnes âgées non-dépendantes, ou ne présentent qu'un faible niveau de dépendance et/ou appartenant au sexe féminin.

Dans le cadre de la mise en oeuvre de la présente invention, ladite souche de *L. casei* peut être utilisée seule, ou en association avec d'autres bactéries lactiques de l'espèce *L*. *casei* ou d'autres espèces. Avantageusement, elle peut être utilisée en association avec des ferments du yogourt, à savoir *Lactobacillus bulgaricus* et *Streptococcus thermophilus.*

De préférence, une composition préparée dans le cadre d'une utilisation conforme à l'Invention contient au moins 10⁵, de préférence au moins 10⁶, généralement entre 1×10⁸ et 1,5×10⁹ cellules de *L. casei* par ml.

Lorsque *L. casei* est utilisé en association avec des ferments du yogourt, ladite composition comprend en outre avantageusement au moins 10⁷, de préférence entre 2×10⁸ et 1×10⁹ cellules de *S. thermophilus* par ml, et au moins 5×10⁵ et de préférence entre 4×10⁶ et 2×10⁷ cellules de *L. bulgaricus* par ml.

Une souche de *L. casei* convenant tout particulièrement à une utilisation dans la présente invention est la souche déposée le 30 décembre 1994, auprès de la CNCM (Collection Nationale de Cultures de Microorganismes, 25 rue du Docteur Roux, Paris), sous le numéro I-1518.

Des compositions préparées conformément à l'invention peuvent être administrées sous forme d'aliments ou de compléments alimentaires. Il peut s'agir par exemple de produits laitiers, et notamment de produits laitiers fermentés comprenant au moins ladite souche de *L. casei,* éventuellement associée, comme indiqué ci-dessus, à d'autres bactéries lactiques, par exemple à des ferments du yogourt.

Ladite souche de *L. casei* sera de préférence administrée pendant au moins une semaine, de préférence pendant au moins 2 semaines, avantageusement pendant au moins 3 semaines, et de manière tout à fait préférée pendant au moins 4 semaines avant la vaccination antigrippale. L'administration de *L. casei* peut ensuite être poursuivie aussi longtemps que souhaité pour maintenir le renforcement de l'immunité induite par la vaccination. La quantité de *L. casei* administrée quotidiennement sera de préférence d'au moins 10¹⁰, avantageusement d'au moins 2 x 10¹⁰ U.F.C. de *L. casei.* Cette quantité peut être administrée en une ou plusieurs prises quotidiennes.

La présente invention sera mieux comprise à l'aide du complément de description qui va suivre, qui se réfère à un exemple non-limitatif illustrant les propriétés d'une souche de *Lactobacillus casei* pour renforcer l'immunité humorale induite par une vaccination anti-grippale.

### EXEMPLES

Une étude a été effectuée afin d'évaluer l'effet de la consommation d'un produit laitier fermenté (Actimel®) contenant *L. casei* CNCM I-1518, sur les taux d'anticorps sériques spécifiques induits par la vaccination anti-grippale 3 semaines après vaccination (pic de la réponse entre 2 à 4 semaines post-vaccination) et 3 et 5 mois après vaccination (pour suivre l'évolution des taux d'anticorps sériques)en cessant la consommation du produit.

### Résumé de l'étude

Cette étude est une étude pilote, multicentrique, randomisée, en double aveugle, contrôlée contre placebo. Les 86 sujets ont été répartis en 2 groupes équilibrés de 43 sujets, un groupe recevant Actimel®, l'autre groupe recevant le produit contrôle (placebo).

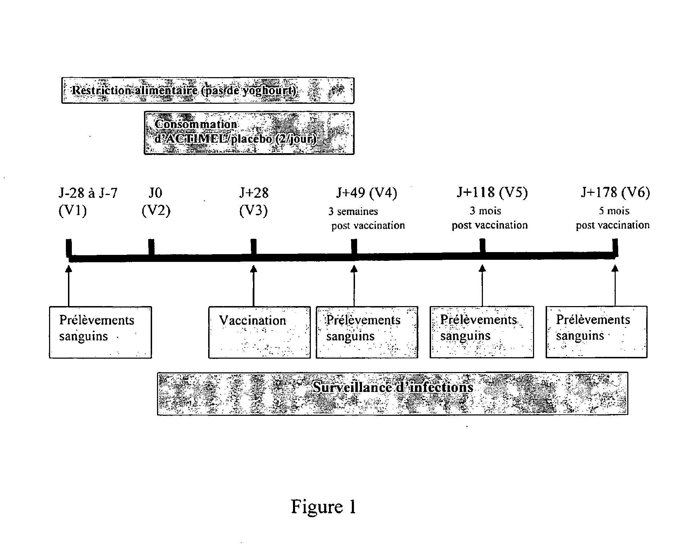

Le déroulement de cette étude est schématisé sur la Figure 1.

La durée totale de l'étude a été de 178 jours.

L'étude a comporté une visite de sélection (V1) visant à sélectionner le sujet âgé. Cette visite a eu lieu dans le mois précédant la visite d'inclusion (V2). L'inclusion et la randomisation des sujets à V2 (J0) a été effectuée sur 2 semaines pour tous les sujets de tous les centres.

La consommation du produit à l'étude (Actimel® ou produit contrôle selon la randomisation) a débuté pour tous les sujets à J0 (V2) et a duré 7 semaines (4 semaines avant vaccination et 3 semaines après).

Les sujets ont été tous vaccinés contre la grippe (même lot du vaccin Vaxigrip) par injection intramusculaire dans le deltoïde à J28 (V3), soit 4 semaines après le début de la consommation du produit à l'étude.

Des visites médicales ont été effectuées à J28 (V3), J49 (V4), J118 (V5), et J178 (V6).

### Sélection des sujets

Les sujets inclus dans l'étude sont des personnes âgées résidant en Établissement d'Hébergement pour Personnes Âgées Dépendantes (EHPAD).

### Critères d'inclusion

- homme ou femme âgé(e) d'au moins 70 ans ;
- sujet ayant un score AGGIR compris entre 2 et 5 (bornes incluses) ;
- sujet ayant un taux d'anticorps inhibant l'agglutination de globules rouges de cobaye par une souche apparentée au variant A/California/7/2004 (prototype vaccinal) inférieur à 40 lors du prélèvement sanguin réalisé à la sélection (V1) ;
- sujet ayant un indice de masse corporelle (IMC) compris entre 16 et 27kg/m² (bornes incluses) ;
- sujet ayant donné son consentement écrit pour sa participation à l'étude et en état de comprendre les informations fournies ;

### Critères de non-inclusion

- sujet atteint d'une pathologie grave et évolutive pour lequel l'espérance de vie attendue est inférieure à 6 mois ;
- sujet diabétique de type I ou II non équilibré ;
- sujet présentant une allergie ou une hypersensibilité aux protéines du lait et/ou aux oeufs ;
- sujet présentant une intolérance connue au lactose ;
- sujet immuno-déprimé chronique ou iatrogène notamment traité par immunosuppresseur ou corticothérapie orale pendant plus de 2 semaines au cours des deux mois précédant la sélection (V1) ;

### Randomisation

L'allocation des sujets au groupe "Produit" ou au groupe "Contrôle" a été définie par un tirage au sort aléatoire, équilibré entre les deux groupes qui ont été identifiés « A » ou « B ».

Deux niveaux de stratification ont été déterminés suivant l'état d'autonomie des patients (reflétant aussi l'état physiologique des sujets) : les sujets ayant un score AGGIR (Autonomie Gérontologique Groupes Iso Ressources) égal à 2 ou 3 constituent la première strate et les sujets ayant un score AGGIR de 4 ou 5 constituent la seconde strate.

### Produits à l'étude

Le produit Actimel® est un produit du commerce.

Le produit contrôle est un produit présentant les mêmes qualités organoleptiques, la même acidité, la même valeur énergétique, et la même texture que le produit Actimel®, mais dépourvu de ferments lactiques. Les 2 produits ont été présentés en bouteilles anonymes de 100 ml, et identifiés par un code lettre (A ou B).

Les caractéristiques de ces deux produits sont résumées dans le Tableau I ci-dessous.

**Tableau I**

| Produit | Lipides g/100g | Glucides G/100g | Protéines G/100g | Energie KJ/100g | Concentration en principe actif (L. casei) |
|---|---|---|---|---|---|
| Actimel® | 1.5 +/- 0.5 | 14.5 +/- 0.5 | 2.5 +/- 0.5 | 80 - 85 | > 10⁸ ufc/ml |
| Contrôle | 1.5 +/- 0.5 | 14.5 +/- 0.5 | 2.5 +/- 0.5 | 80-85 | <1ufc/ml |

### Déroulement de l'étude

A l'issue de la visite de sélection, les sujets présentant tous les critères d'inclusion et aucun des critères de non-inclusion définis ci-dessus ont été retenus.

Il leur a été demandé de s'abstenir de consommer des produits laitiers fermentés (yaourts, fromage blanc, petits suisses et yaourts au soja), au cours des 7 jours précédant le début de l'étude (entre J-7 et J0) et jusqu'à la visite V4 (J49)

Un prélèvement sanguin a été réalisé afin de doser le taux basal d'anticorps contre la grippe.

A J0, la visite d'inclusion et de randomisation (V2) a été effectuée.

A partir de J0, les sujets ont consommé tous les jours le produit à l'étude (A ou B) attribué par la randomisation à raison d'une bouteille de 100 ml lors du petit-déjeuner, et une bouteille de 100 ml lors du dîner, pendant 49 jours consécutifs.

A J28 (V3), les sujets ont été vaccinés contre la grippe par injection intramusculaire dans le deltoïde. Le vaccin utilisé (Vaxigrip), est un vaccin inactivé à virion fragmenté, dont chaque dose contient 15µg d'hémagglutinine de chacune des souches suivantes : 2 souches de type A (1 souche H1N1 et 1 souche H3N2), et 1 souche de type B. 15µg d'hémagglutinine par dose de chacune des souches décrites ci-dessus. Ce vaccin ne contient pas d'adjuvant.

La souche H1N1 est la souche New Caledonia/20/99 ; la souche H3N2 est la souche California/7/2004, et la souche B est la souche Shangai/361/2002 (selon les recommandations de l'OMS pour la vaccination dans l'hémisphère Nord lors de la saison 2005-2006).

A chacune des visites suivantes, effectuées à J49 (V4), J118 (V5), et J178 (V6), et un prélèvement sanguin a été réalisé afin de doser les anticorps sériques spécifiques de chacune des souches présentes dans le vaccin, induits par la vaccination anti-grippale. Un examen clinique a également été réalisé, et les pathologies ou prises de traitement survenues depuis la visite précédente ont à chaque fois été notées.

### Dosage des anticorps

Des prélèvements sanguins (1 tube de 7 ml) ont été effectués dans une veine de l'avant-bras chez l'ensemble des sujets lors des visites V1, V4 et V5. Les prélèvements ont été faits sur tubes secs, puis centrifugés pour séparer le sérum. Chaque sérum a été aliquoté dans des cryotubes (500µl par tube) pour être conservé entre -20°c et -80°c jusqu'au moment des dosages. Ces dosages ont été réalisés par le centre national de référence des virus Influenza (CNR); Institut Pasteur, Paris, France.

Le test utilisé est le test d'inhibition de l'hémagglutination (IHA), effectué selon les recommandations OMS (WHO Manual on Animal Influenza Diagnosis and Surveillance :WHO/CDS/CSR/NCS/2002-5). Ce test est basé sur la capacité des anti-corps anti-hémagglutinine spécifiques de chaque souche virale qui sont contenus dans le sérum testé, à se fixer à l'hémagglutinine exprimée à la surface des virus et à empêcher ainsi la liaison de ces virus aux globules rouges. En l'absence d'anticorps spécifiques, on observe la formation d'un réseau entre les globules rouges et le virus (puits de culture uniformément rouge). En revanche, en présence d'anticorps spécifiques, on observe une sédimentation des globules rouges au fond du puits.

Chaque sérum a été traité par incubation avec du RDE (Receptor destroying enzyme) puis absorbé sur des globules rouges de coq afin d'éliminer une agglutination non spécifique due au sérum et non au virus grippal. Chaque sérum a été dilué de 2 en 2, réparti dans des plaques de culture et incubé avec une suspension virale standardisée (4 unités hémagglutinantes pour 50 µl). Des globules rouges de cobaye ont ensuite été ajoutés, pour révéler la présence d'hémagglutinine virale non neutralisée par les anticorps.

Chaque test a été validé par le dosage simultané de contrôles positifs et négatifs provenant du CNR pour le test d'inhibition de l'hémagglutination.

Les titres d'anticorps sont exprimés par l'inverse de la dilution la plus élevée donnant encore une inhibition de l'hémagglutination.

### ANALYSES STATISTIQUES

L'analyse des données a été effectuée sur les populations et sous-populations suivantes :
- Population "Intention To Treat" (ITT) comprenant tous les sujets inclus dans l'étude, randomisés et ayant reçu au moins un des produits étudiés;
- Sous-populations, selon la strate AGGIR et selon le sexe.

Les tests ont été effectués au seuil de significativité de 5% en bilatéral. Un seuil de significativité compris entre 5 et 10 % a été considéré comme indicatif d'une tendance.

Le plan expérimental est celui d'un groupe parallèle avec 2 groupes "Produit" versus "Contrôle".

Compte-tenu des objectifs de l'étude, l'effet principal de cette étude est le groupe « produit ».

Les effets du « score AGGIR », de l'IMC, de l'âge et du taux basal d'anticorps ont été également étudiés.

Pour le taux d'anticorps, il est connu que ce paramètre est distribué selon une loi log-normal. Pour faire une analyse paramétrique, il est nécessaire de faire une transformation log sur les taux d'anticorps et la normalité des log de taux d'anticorps a donc été vérifiée et discutée.

L'analyse paramétrique est un modèle gaussien usuel d'analyse de la variance et/ou covariance selon la nature des covariables.

L'analyse de variance et/ou covariance non paramétrique (test de Friedman) est effectuée conjointement.

Les co-variables suivantes ont été prises en compte dans les analyses : « score AGGIR », de l'IMC, de l'âge et du taux basal d'anticorps.

La comparaison des données entre les produits a été faite par un test du Chi-2 ou par un test exact de Fisher en cas de non respect des conditions d'utilisation du test du chi-2. Un modèle de régression logistique a également été réalisé pour la prise en compte des différents effets (:« score AGGIR », de l'IMC, de l'âge et du taux basal d'anticorps).

Cette même analyse a également été réalisée pour chaque classe de la variable de stratification (« Score AGGIR »).

### Critères de jugement

### Critère principal d'effet du produit étudié

Le critère principal retenu est le taux d'anticorps de la grippe à la visite 4 (J49) sur les 3 souches virales.

L'expression principale de ce critère est la variation du taux d'anticorps à la visite 4 par rapport à la valeur basale.

### Critères secondaires d'effet du produit étudié

Les critères secondaires retenus sont, pour chacune des 3 souches virales :
- La séroconversion (sujet ayant une augmentation d'au moins 4 fois du taux d'anticorps par rapport au niveau basal) à la visite 4 (J49).
- La séroprotection (sujet ayant un taux d'anticorps ≥ 40 ) à la visite 4 (J49).

### RESULTATS

### Sur la population ITT

### Taux d'anticorps

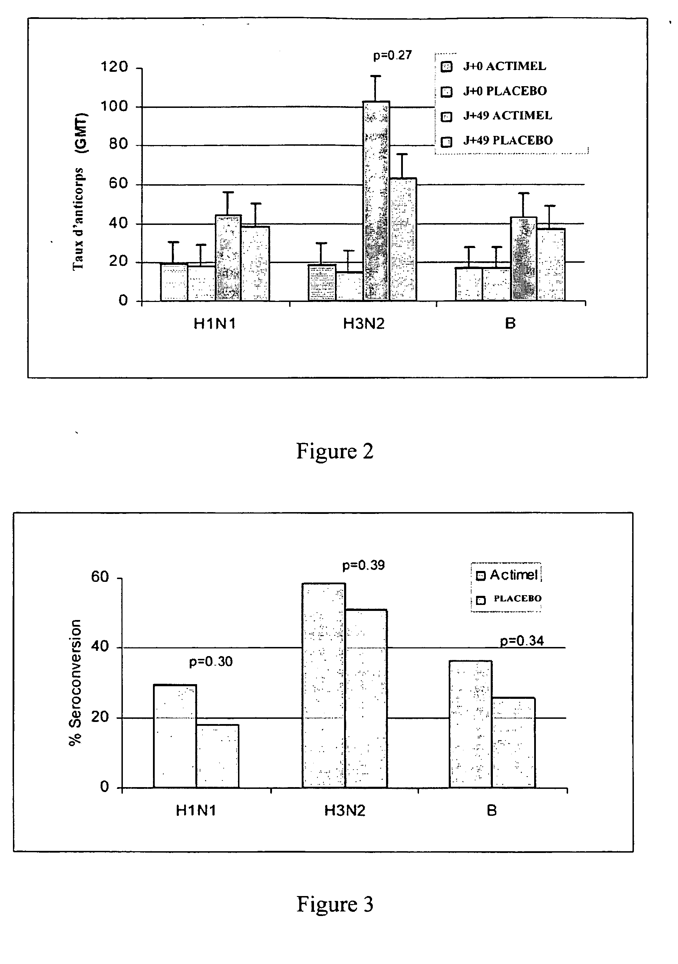

Les résultats sont illustrés par la Figure 2. Ces résultats montrent que la consommation d'Actimel induit, 3 semaines après vaccination contre chacune des souches de la grippe un taux d'anticorps supérieur à celui relevé dans le cas de la consommation du placebo.

### Séroconversion

Les résultats sont illustrés par la Figure 3. Ces résultats montrent que la consommation d'Actimel induit 3 semaines après vaccination, une fréquence de séroconversion plus importante vis-à vis de chacune des souches de la grippe, que celle observée dans le cas de la consommation du placebo.

### Séroprotection

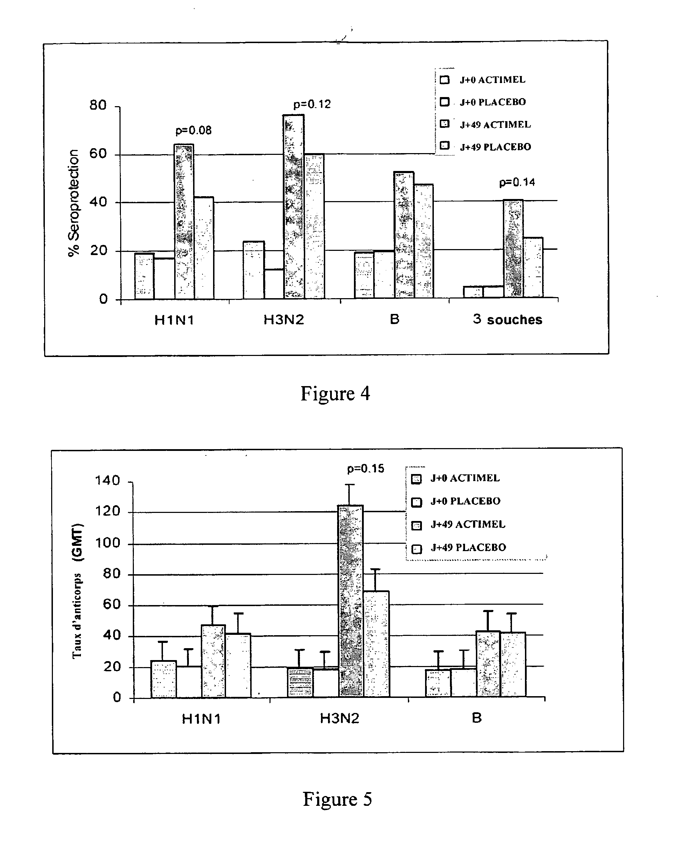

Les résultats sont illustrés par la Figure 4. Ces résultats montrent que la consommation d'Actimel induit, 3 semaines après vaccination contre chacune des souches de la grippe une augmentation du pourcentage de personnes protégées, par rapport à celui observé dans le cas de la consommation du placebo. Cet effet montre une tendance statistique dans le cas de la souche de sérotype H1N1.

### Sur les sous populations

### Sous population AGGIR 4-5

### Taux d'anticorps

Les résultats sont illustrés par la Figure 5. Ces résultats montrent que la consommation d'Actimel induit, 3 semaines après vaccination contre chacune des souches de la grippe un taux d'anticorps supérieur à celui relevé dans le cas de la consommation du placebo.

### Séroconversion

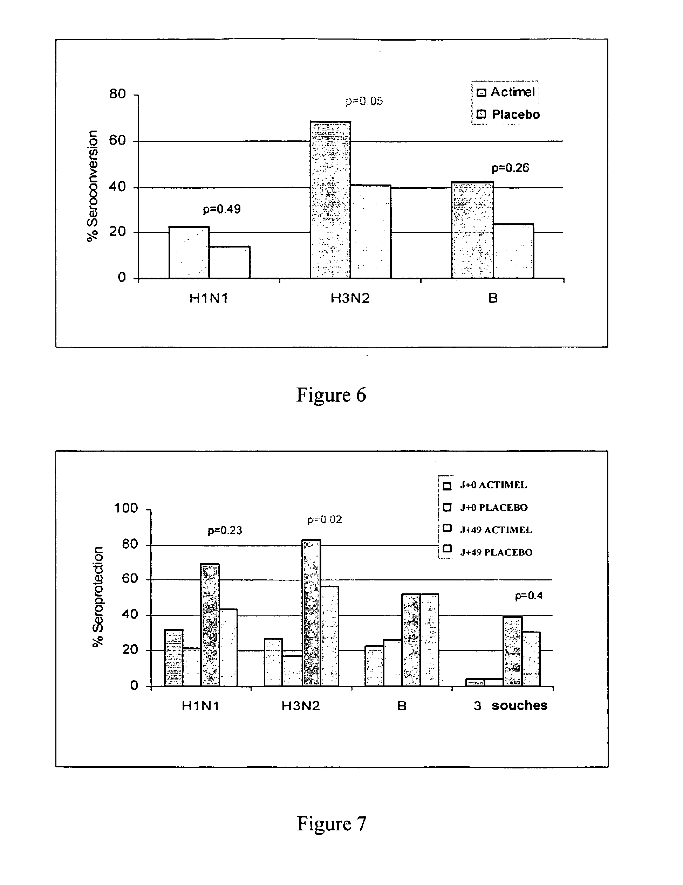

Les résultats sont illustrés par la Figure 6. Ces résultats montrent que la consommation d'Actimel induit 3 semaines après vaccination, une fréquence de séroconversion plus importante vis-à vis de chacune des souches de la grippe, que celle observée dans le cas de la consommation du placebo. Cet effet est statistiquement significatif pour la souche de sérotype H3N2.

### Séroprotection

Les résultats sont illustrés par la Figure 7. Ces résultats montrent que la consommation d'Actimel induit, 3 semaines après vaccination contre chacune des souches de la grippe une augmentation du pourcentage de personnes protégées, par rapport à celui observé dans le cas de la consommation du placebo. Cet effet est statistiquement significatif pour la souche de sérotype H3N2.

### Sous-population de sexe féminin

### Taux d'anticorps

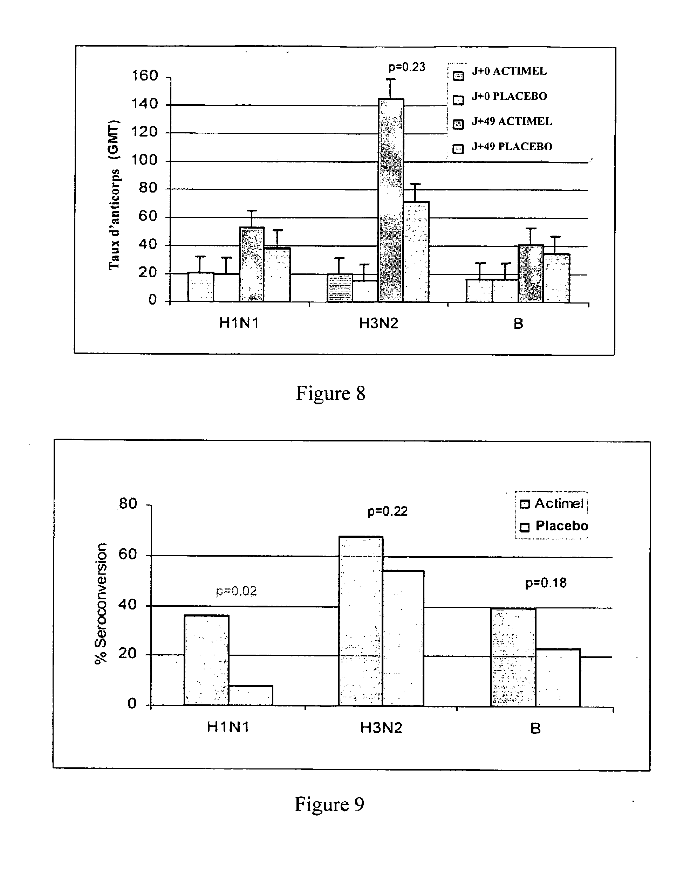

Les résultats sont illustrés par la Figure 8. Ces résultats montrent que la consommation d'Actimel induit, 3 semaines après vaccination contre chacune des souches de la grippe un taux d'anticorps supérieur à celui relevé dans le cas de la consommation du placebo.

### Séroconversion

Les résultats sont illustrés par la Figure 9. Ces résultats montrent que la consommation d'Actimel induit 3 semaines après vaccination, une fréquence de séroconversion plus importante vis-à vis de chacune des souches de la grippe, que celle observée dans le cas de la consommation du placebo. Cet effet est statistiquement significatif dans le cas de la souche de sérotype H1N1.

### Séroprotection

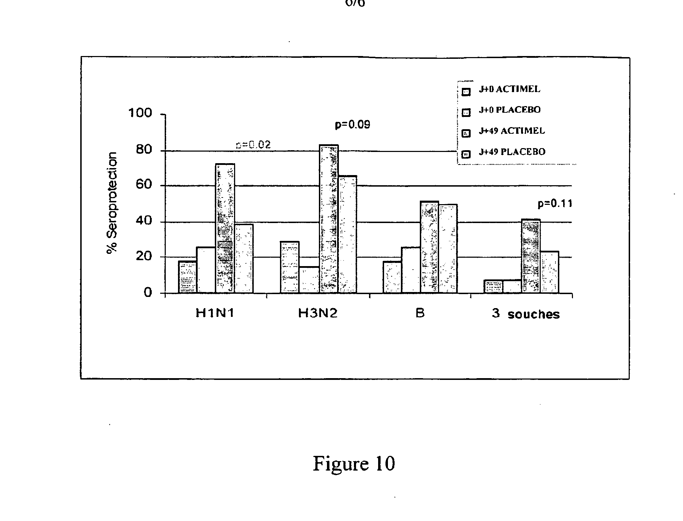

Les résultats sont illustrés par la Figure 10. Ces résultats montrent que la consommation d'Actimel induit, 3 semaines après vaccination contre chacune des souches de la grippe une augmentation du pourcentage de personnes protégées, par rapport à celui observé dans le cas de la consommation du placebo. Cet effet est plus accentué dans le cas des souches du groupe A (tendance statistique pour la souche H3N2), et plus particulièrement pour la souche de sérotype H1N1 (différence statistiquement significative).

## Revendications

1. Utilisation d'une souche bactérienne de l'espèce *L. casei* pour la préparation d'une composition administrable par voie orale pour renforcer l'immunité humorale conférée lors d'une vaccination anti-grippale, ladite souche étant administrée pendant au moins 1 semaine avant ladite vaccination anti-grippale.

2. Utilisation selon la revendication 1, **caractérisée en ce que** ladite souche est administrée pendant au moins 4 semaines avant ladite vaccination anti-grippale.

3. Utilisation selon une quelconque des revendications 1 ou 2, **caractérisée en ce que** ladite composition est destinée à être administrée à des personnes âgées de 65 ans ou plus.

4. Utilisation selon une quelconque des revendications 1 à 3, **caractérisé en ce que** ladite souche de *L. casei* est la souche CNCM 1-1518.

5. Utilisation selon une quelconque des revendications 1 à 4, **caractérisée en ce que** ladite composition est sous la forme d'un aliment ou d'un complément alimentaire.

6. Utilisation selon une quelconque des revendications 1 à 5, **caractérisée en ce que** ladite composition est sous la forme d'un produit laitier fermenté.

7. Utilisation selon une quelconque des revendications 1 à 6, **caractérisée en ce que** ladite souche est administrée quotidiennement à une quantité d'au moins 10¹⁰ U.F.C. de *L. casei.*

8. Utilisation selon une quelconque des revendications 1 à 7, caractérisée en ce ladite composition contient au moins 10⁵, de préférence au moins 10⁶, de préférence encore entre 1.10⁸ et 1,5.10⁹ cellules de *L. casei* par ml.

## Claims

1. Use of a bacterial strain of the species *L. casei* for preparing a composition which can be administered orally with a view to strengthening the humoral immunity imparted during an anti-influenza vaccination, said strain being administered for at least 1 week prior to said anti-influenza vaccination.

2. Use as claimed in claim 1, **characterised in that** said strain is administered for at least 4 weeks prior to said anti-influenza vaccination.

3. Use as claimed in any one of claims 1 or 2, **characterised in that** said composition is intended to be administered to persons aged 65 years or more.

4. Use as claimed in any one of claims I to 3, **characterised in that** said strain of *L. casei* is the CNCM I-1518 strain.

5. Use as claimed in any one of claims I to 4, **characterised in that** said composition is in the form of a foodstuff or a food supplement.

6. Use as claimed in any one of claims 1 to 5, **characterised in that** said composition is in the form of a fermented dairy product.

7. Use as claimed in any one of claims I to 6, **characterised in that** said strain is administered daily in a quantity of at least 10¹⁰ U.F.C. of *L. casei.*

8. Use as claimed in any one of claims I to 7, **characterised in that** said composition contains at least 10⁵, preferably at least 10⁶, even more preferably between 1.10⁸ and 1.5.10⁹, cells of *L. casei* per ml..

## Patentansprüche

1. Verwendung eines Bakterienstammes der Art *L. casei* zur Herstellung einer Zusammensetzung zur oralen Verabreichung, um die humorale Immunität, verliehen bei einer Anti-Grippe-Impfung, zu verstärken, wobei der Stamm während mindestens einer Woche vor der Anti-Grippe-Impfung verabreicht wird.

2. Verwendung gemäß Anspruch 1, dadurch gekennzeichn e t , dass der Stamm mindestens während vier Wochen vor der Anti-Grippe-Impfung verabreicht wird.

3. Verwendung gemäß einem der Ansprüche 1 oder 2, **dadurch gekennzeichnet, dass** die Zusammensetzung zur Verabreichung an Personen im Alter von 65 Jahren oder mehr bestimmt ist.

4. Verwendung gemäß einem der Ansprüche 1 bis 3, **dadurch gekennzeichnet, dass** der *L. casei*-Stamm der Stamm CNCM 1-1518 ist.

5. Verwendung gemäß einem der Ansprüche 1 bis 4, **dadurch gekennzeichnet , dass** die Zusammensetzung in der Form eines Nahrungsmittels oder eines Nahrungse.rgänzungsmittels vorliegt.

6. Verwendung gemäß einem der Ansprüche 1 bis 5, **dadurch gekennzeichnet , dass** die Zusammensetzung in der Form eines fermentierten Milchproduktes vorliegt.

7. Verwendung gemäß einem der Ansprüche 1. bis 6, **dadurch gekennzeichnet, dass** der Stamm täglich in einer Menge von mindestens 10¹⁰ U.F.C. an *L*. *casei* verabreicht wird.

8. Verwendung gemäß einem der Ansprüche 1 bis 7, **dadurch gekennzeichnet, dass** die Zusammensetzung mindestens 10⁵, bevorzugt mindestens 10⁶, weiter bevorzugt zwischen 1.10⁸ und 1,5.10⁹ Zellen von *L. casei* pro ml enthält.
